# EUROPÄISCHE PATENTANMELDUNG

(11) **EP 4 098 292 A1**
(43) Veröffentlichungstag der Anmeldung: **07.12.2022**
(21) Anmeldenummer: 21177303.1
(22) Anmeldetag: 02.06.2021
(51) Int. Cl.: A61M 1/36, B01D 63/06, B01D 69/04

(54) **BLUTFILTERELEMENT UND VERFAHREN ZU SEINER HERSTELLUNG**

(71) Anmelder: Sefar AG, 9410 Heiden (CH)
(72) Erfinder: Dür, Hansjörg, 6858 Schwarzach (AT)
(74) Vertreter: Wunderlich & Heim Patentanwälte Partnerschaftsgesellschaft mbB

(57) **Zusammenfassung**

Die Erfindung betrifft ein Blutfilterelement, welches taschen- oder schlauchförmig mit einem sich in einer Längsrichtung erstreckenden Innenraum ausgebildet ist und einen mehrlagigen Aufbau aufweist, welcher eine durchlässige Außenlage, eine durchlässige Innenlage und mindestens eine zwischen der Außenlage und der Innenlage angeordnete Zwischenlage aufweist, welche als ein feinporiges Filterelement ausgebildet ist, wobei das Blutfilterelement eine vorgegebene axiale Länge aufweist, und wobei die Außenlage und die Innenlage die vorgegebene axiale Länge aufweisen. Es ist erfindungsgemäß, dass die Zwischenlage axial kürzer als die Außenlage und die Innenlage unter Ausbildung eines Überlaufbereiches ausgebildet ist, welcher an einem axialen oberen Endbereich des Blutfilterelementes angeordnet ist, wobei eine Oberkante der Zwischenlage von der Oberkante der Außenlage und der Innenlage axial nach unten beabstandet ist, dass die Außenlage, die Innenlage und die Zwischenlage entlang mindestens einem Längsrand unter Ausbildung eines Längsverbindungssaumes fest miteinander verbunden sind, und dass die Außenlage und die Innenlage entlang ihrer Oberkante unter Ausbildung eines oberen Querverbindungssaumes fest miteinander verbunden sind, wobei die Oberkante der Zwischenlage frei und unverbunden zwischen der Außenlage und der Innenlage angeordnet ist.

## Beschreibung

Blutfilterelement, welches taschen- oder schlauchförmig mit einem sich in einer Längsrichtung erstreckenden Innenraum ausgebildet ist und einen mehrlagigen Aufbau aufweist, welcher eine durchlässige Außenlage, eine durchlässige Innenlage und mindestens eine zwischen der Außenlage und der Innenlage angeordnete Zwischenlage aufweist, welche als ein feinporiges Filterelement ausgebildet ist, wobei das Blutfilterelement eine vorgegebene axiale Länge aufweist, und wobei die Außenlage und die Innenlage die vorgegebene axiale Länge aufweisen, gemäß dem Oberbegriff des Anspruchs 1.

Die Erfindung betrifft weiterhin ein Verfahren zum Herstellen eines solchen Blutfilterelementes gemäß dem Anspruch 13.

Derartige Blutfilter werden insbesondere bei sogenannten Herz-Lungen-Maschinen zum Reinigen von zirkulierendem Blut und zum Rückhalten zu großer Gasbläschen im Blut eingesetzt.

Aus der WO 98/15302 A1 ist ein Blutfilterelement mit drei Lagen bekannt, wobei die einzelnen Lagen an einem starren Grundkörper oder Rahmen angebracht sind. Durch den Rahmen wird eine zylindrische Form vorgegeben.

Aus der DE 2 530 413 C3 geht ein beutelförmiger Blutfilter hervor, welcher durch Verschweißen verschiedener Lagen gebildet ist.

Der Erfindung liegt die **Aufgabe** zugrunde, ein einfaches und robust aufgebautes Blutfilterelement und ein Verfahren zu seiner Herstellung anzugeben, mit welchen eine besonders effiziente Blutfiltrierung durchgeführt werden kann.

Die Aufgabe wird zum einen durch ein Blutfilterelement mit den Merkmalen des Anspruchs 1 und zum anderen durch ein Verfahren mit den Merkmalen des Anspruchs 13 gelöst. Bevorzugte Ausführungsformen der Erfindung sind in den abhängigen Ansprüchen angegeben.

Das erfindungsgemäße Blutfilterelement ist dadurch gekennzeichnet, dass die Zwischenlage axial kurzer als die Außenlage und die Innenlage unter Ausbildung eines Überlaufbereiches ausgebildet ist, welcher in einem axialen oberen Endbereich des Blutfilterelementes angeordnet ist, wobei eine Oberkante der Zwischenlage von den Oberkanten der Außenlage und der Innenlage axial nach unten beabstandet ist, dass die Außenlage, die Innenlage und die Zwischenlage entlang mindestens einem Längsrand unter Ausbildung eines Längsverbindungssaumes fest miteinander verbunden sind, und dass die Außenlage und die Innenlage entlang ihrer Oberkante unter Ausbildung eines oberen Querverbindungssaumes fest miteinander verbunden sind, wobei die Oberkante der Zwischenlage frei und unverbunden zwischen der Außenlage und der Innenlage angeordnet ist.

Eine Grundidee der Erfindung liegt darin, dass bei einem mehrlagigen Aufbau eines socken- oder schlauchförmigen Blutfilterelementes das Filterelement als eine Zwischenlage zwischen einer Außenlage und einer Innenlage, jedoch mit einer unterschiedlichen Länge hierzu, ausgebildet wird. Die Zwischenlage wird dabei definiert axial kürzer ausgeführt, so dass eine Oberkante der Zwischenlage unter Ausbildung eines Freiraumes von den Oberkanten der Außenlage und der Innenlage beabstandet ist. In dem oberen Abschnitt des Blutfilterelementes ist somit ein nur zweilagiger Aufbau gegeben, wodurch ein Überlaufbereich gebildet ist. Der Überlaufbereich ohne die Zwischenlage weist einen geringeren Durchflusswiderstand auf. So kann in bestimmten Situationen überschüssiges Blut schnell und effizient über den Überlaufbereich abgeführt werden, wobei durch die Außenlage und die Innenlage eine Mindestfilterwirkung sichergestellt ist.

Ein weiterer Aspekt der Erfindung besteht darin, dass es für eine Lagefixierung der Zwischenlage in dem Blutfilterelement ausreichend ist, wenn eine feste Verbindung mit der Außenlage und der Innenlage zumindest entlang einer Längskante mit einem Längsverbindungssaum besteht. Insbesondere beruht die Erfindung auf der Erkenntnis, dass eine Oberkante der Zwischenlage frei und unverbunden bleiben kann. Insbesondere kann so bei der Herstellung des Blutfilterelementes auf den Einsatz zusätzlicher Schmelzfolien zur Verbindung innenliegender Lagen verzichtet werden. Durch ein Verbinden der Zwischenlage entlang einer Längsrandes mit den beiden anderen Lagen und einer Verbindung zwischen der Außenlage und der Innenlage an der Oberkante wird ein hinreichend robuster Aufbau erreicht. Durch eine geringe Anzahl von Verbindungssäumen wird eine hohe und wirksame Filterfläche erreicht.

Somit kann bei einer effizienten Fertigung ein besonders vorteilhafter und funktionsfähiger Aufbau eines Blutfilterelementes erzielt werden.

Das erfindungsgemäße Blutfilterelement wird grundsätzlich so eingebaut, dass eine Anströmung des zu reinigenden Blutes in den Innenraum von oben oder vorne erfolgt. Zum Reinigen tritt das Blut radial durch die etwa hohlzylindrische Umfangswand des Blutfilterelementes nach außen. Das Blutfilterelement kann dabei schlauchförmig mit einer oberen und einer unteren Öffnung versehen sein oder alternativ eine Taschen- oder Sockenform aufweisen, bei welcher eine untere Öffnung verschlossen ist. Das Blutfilterelement wird dabei insgesamt so betrieben, dass in einem normalen Filterbetrieb das zu reinigende Blut durch den unteren und mittleren Bereich, welcher dreilagig ausgebildet ist, durch die Wandung des Blutfilterelementes hindurchtritt. Durch eine entsprechende Ausbildung der inneren Lage kann eine zusätzliche Filterwirkung, insbesondere eine Kaskadenfiltration zusammen mit der Zwischenlage erzielt werden. Tritt ein unerwartetes Zusetzen oder eine Schaumbildung im Blut in diesem Bereich auf, kann ein Übertritt von Blut auch über den zweilagigen Überlaufbereich radial nach außen erfolgen. Hierdurch wird ein ausreichender Blutkreislauf, etwa durch eine Herz-Lungen-Maschine und damit für den Patienten sichergestellt.

Grundsätzlich kann das zu reinigende Blut auch bei einer alternativen Ausgestaltung radial von außen nach innen fließen.

Eine bevorzugte Ausführungsform der Erfindung besteht darin, dass die Außenlage, die Innenlage und/oder die Zwischenlage an ihrer Unterkante unter Ausbildung eines unteren Querverbindungssaumes fest miteinander verbunden sind. Dabei können jeweils nur zwei Lagen oder alle Lagen ringförmig miteinander verbunden sein. Hierdurch wird die Stabilität des Aufbaus erhöht. Für ein taschenförmiges Filterelement ist der untere Querverbindungssaum so ausgebildet, dass eine Öffnung des Innenraumes nach unten vollständig verschlossen ist.

Allgemein kann das erfindungsgemäße Filterelement so aufgebaut sein, dass ein Ausgangsmaterial mit den drei übereinander angeordneten Lagen, welche entsprechend der gewünschten Länge und einer doppelten Breite konfektioniert sind, einmal gefaltet und übereinandergeschlagen werden. Zum Bilden des insbesondere schlauchförmigen Filterelementes kann dann ein festes Verbinden entlang der Längskante erfolgen, so dass ein Blutfilterelement mit nur einem Längsverbindungssaum gebildet ist. Die Schlauchform kann zylindrisch oder konisch sein.

Eine besonders stabile Ausgestaltung der Erfindung besteht darin, dass ein Aufbau aus zwei Hälften vorgesehen ist und dass die zwei Hälften entlang der beiden Längsränder unter Ausbildung von zwei Längsverbindungssäumen fest miteinander verbunden sind. Die jeweiligen Hälften können dabei gleich konfektioniert werden und weisen jeweils die drei Lagen auf. Dabei ist erfindungsgemäße die Zwischenlage steht definiert axial kürzer als die Außenlage und die Innenlage.

Eine weitere bevorzugte Ausführungsform der Erfindung besteht darin, dass eine axiale Länge des Überlaufbereiches zwischen 5% bis 30% der axialen Länge des Blutfilterelementes beträgt. In dem eigentlichen Filterbereich ist eine Seitenwand des Filterelementes dreilagig ausgebildet, während in dem Überlaufbereich ein zweilagiger Aufbau aus der Außenlage und der Innenlage ohne Zwischenlage gebildet ist.

Grundsätzlich kann ein Verbinden der Lagen mit jedem geeigneten Verbindungsverfahren erfolgen. Fertigungstechnisch besonders vorteilhaft ist es, dass mindestens ein Verbindungssaum durch Ultraschallverschweißen gebildet ist. Dadurch lassen sich insbesondere Polymermaterialien mit einer geringen Verbindungsbreite effizient und zuverlässig bei einem schmalen und sauberen Verbindungssaum fest miteinander verbinden.

Zum Bilden eines taschenförmigen Blutfilterelementes ist es nach einer Ausführungsvariante der Erfindung vorgesehen, dass der untere Querverbindungssaum zum Bilden eines geschlossenen Bodens ausgebildet ist. Hierdurch wird ein freier Durchtritt von Blut nach unten aus dem Blutfilterelement heraus unterbunden. Es kann so eine Reinigung des gesamten Blutes sichergestellt werden, welches in das Blutfilterelement eintritt.

Generell kann die feinporige Zwischenlage zum Filtern in jeder geeigneten Weise ausgebildet sein. Besonders zweckmäßig ist es nach einer Weiterbildung der Erfindung, dass das Filterelement ein Filtergewebe, eine Filtermembran, ein Filtergewirk und/oder ein Filtervlies aufweist. Entsprechend der vorgesehenen Filtrationsaufgabe sind eine Poren- oder Öffnungsgröße, ein Öffnungsanteil an der Filterfläche sowie eine Filterleistung oder ein Durchflusswiderstand auszuwählen.

Besonders vorteilhaft ist es nach einer Weiterbildung der Erfindung, dass eine Öffnungs- oder Porengröße des Filterelementes zwischen 10 µm und 400 µm liegt, insbesondere zwischen 25 µm und 60 µm. Dies kann insbesondere durch die Ausbildung des Filterelementes als ein Filtergewebe erreicht werden, wobei vorzugsweise ein Öffnungsanteil zwischen 30% bis 60% an der Gesamtfilterfläche erreicht werden kann. Dies ist insbesondere durch die Verwendung sehr feiner Fäden möglich, die vorzugsweise zwischen 10 µm bis 30 µm, besonders bevorzugte zwischen 18 µm bis 28 µm betragen.

Grundsätzlich weisen die Außenlage und die Innenlage eine durchlässige Struktur auf, insbesondere mit einer größeren Öffnungs- oder Porengröße wie die Zwischenlage. Nach einer Ausbildungsvariante der Erfindung ist es vorteilhaft, dass die Außenlage und/oder die Innenlage ausgebildet ist als ein Gewebe, ein Gewirk, Netz, Gitter und/oder Vlies. Besonders zweckmäßig ist der Einsatz eines Gewebes oder eines Gewirkes, welches vorzugsweise mit monofilen bzw. multifilen Fäden gebildet ist. Hierdurch wird ein Lösen von Teilen aus der Lage weitgehend unterbunden.

Eine besonders gute Reinigungswirkung wird dadurch erzielt, dass das Material der Zwischenlage, der Außenlage und der Innenlage ein Polymermaterial ist, insbesondere Polyester oder Polyamid. Bei dem Material handelt es sich um ein medizinisch zugelassenes, besonders reines Polymermaterial.

Nach einer Weiterbildung der Erfindung ist es zudem besonders zweckmäßig, dass das Material der Zwischenlage, der Außenlage und der Innenlage gleich ist. Die Verwendung des gleichen Materials verbessert die Anwendbarkeit im medizinischen Sektor und erleichtert auch eine Zulassung als medizinisches Produkt. Insbesondere in Kombination mit einem Verschweißen, insbesondere einem Ultraschallverschweißen, kann ein sortenreines Blutfilterelement gebildet werden, welches frei von zusätzlichen Klebstoffen oder anderen Materialien ist. Hierdurch kann eine besonders hohe Produktsicherheit gewährleistet werden.

Eine weitere vorteilhafte Ausführungsform der Erfindung besteht darin, dass eine Öffnungsgröße der Außenlage und/0der der Innenlage zwischen 100 µm und 400 µm beträgt, insbesondere zwischen 150 µm und 300 µm. Hierdurch wird ein Durchflusswiderstand in dem Blutfilterelement in Bezug auf die maßgeblich filternde Zwischenlage kaum beeinträchtigt. Zudem wird mit diesem Größenbereich insbesondere bei einem Öffnungsanteil von über 50% in der Außenlage und der Innenlage, eine gute Überlauffunktion für den Überlaufbereich erreicht.

Bezüglich des Verfahrens zum Herstellen eines Blutfilterelementes ist die Erfindung dadurch gekennzeichnet, dass die Außenlage und die Innenlage an ihren Oberkanten unter Ausbildung eines oberen Querverbindungssaumes fest miteinander verbunden werden, wobei die Zwischenlage in einem Zwischenraum zwischen der Außenlage und der Innenlage angeordnet ist und eine Oberkante der Zwischenlage frei und ungebunden bleibt. Hierdurch wird ein Blutfilterelement mit einer Überlauffunktion in einem oberen Bereich durch eine nur Zweilagenaufbau erreicht, wobei eine Oberkante der filtrierenden Zwischenlage frei und ungebunden zwischen der Außenlage und der Innenlage verbleibt.

Durch das Verfahren kann insbesondere das zuvor beschriebene Blutfilterelement effizient hergestellt werden, wobei die zuvor beschriebenen Vorteile erreicht werden.

Eine besonders gute Verbindung zwischen den einzelnen flexiblen Lagen kann nach einer Weiterbildung der Erfindung dadurch bereitgestellt werden, dass ein Verbinden der Außenlage, der Innenlage und/oder der Zwischenlage an ihren Außenrändern über ein Ultraschallverschweißen, ein thermisches Verschweißen, ein Kleben und/oder ein Heißkleben erfolgt. Insbesondere bei einem Ultraschallverschweißen und bei einem thermischen Verschweißen kann der Einsatz von Klebstoffen oder zusätzlichen Komponenten entfallen.

Nach einer weiteren Verfahrensvariante der Erfindung ist es zweckmäßig, dass durch ein Verbinden der flexiblen Lagen eine taschen- oder schlauchförmige Zwischenform mit außenliegenden Verbindungssäumen gebildet wird, und dass die Zwischenform zum Bilden des Filterelementes gestülpt wird, wobei die Verbindungssäume nach innen gedreht werden. Hierdurch kann eine geschützte Anordnung der Verbindungssäume am Zwischenprodukt erzielt werden. Bei einer Anströmung des Blutfilterelementes von innen wird zudem sichergestellt, dass sich selbst bei einem Lösen kleinster Bestandteile aus dem Verbindungssaum diese nicht in den eigentlichen Blutkreislauf gelangen können, da sie dann von der in Strömungsrichtung nachfolgenden filtrierenden Zwischenlage aufgefangen werden.

Die Erfindung wird nachfolgend anhand von bevorzugten Ausführungen weiter beschrieben, welche schematisch in den Zeichnungen dargestellt sind. In den Zeichnungen zeigen:
- Fig. 1: eine Seitenansicht eines ersten erfindungsgemäßen Filterlementes;
- Fig. 2: eine Querschnittsansicht des Filterelementes von Fig. 1;
- Fig. 3: eine Draufsicht von oben auf das Blutfilterelement nach den Figuren 1 und 2;
- Fig. 4: eine vergrößerte Darstellung des Details A von Fig. 2;
- Fig. 5: eine vergrößerte Darstellung des Details B von Fig. 2;
- Fig. 6: eine Seitenansicht eines zweiten erfindungsgemäßen Blutfilterelementes;
- Fig. 7: eine Querschnittsansicht des Filterelementes von Fig. 6;
- Fig. 8: eine Draufsicht von oben auf das Blutfilterelement nach den Figuren 6 und 7;
- Fig. 9: eine vergrößerte Darstellung des Details A von Fig. 7; und
- Fig. 10: eine vergrößerte Darstellung des Details B von Fig. 7.

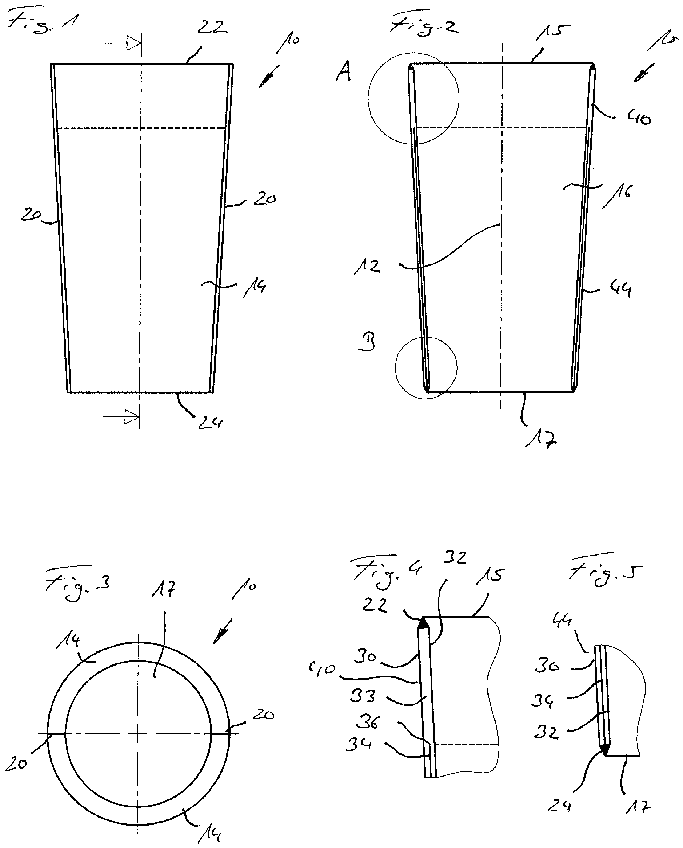

Gemäß den Figuren 1 bis 5 ist ein erstes Ausführungsbeispiel eines erfindungsgemäßen Blutfilterelementes 10 dargestellt, welches schlauchförmig mit einem durchgehenden Innenraum 16 mit einer oberen Öffnung 15 und einer unteren Öffnung 17 ausgebildet ist. Das schlauchförmige Blutfilterelement 10 ist leicht konisch entlang einer Längsachse 12 zur unteren Öffnung 17 hin. Die Öffnungen 15, 17 sind etwa kreisförmig ausgebildet.

Das Blutfilterelement 10 ist aus zwei Hälften 14 gebildet, welche entlang von zwei Längsrändern entlang der Längsachse 12 mit Längsverbindungssäumen 20 miteinander verbunden sind. Das Verbinden kann insbesondere mittels eines Ultraschallverscheißens erfolgen. Jede Hälfte 14 weist drei Lagen auf, nämlich eine Außenlage 30, eine Innenlage 32 und eine dazwischen in einem Zwischenraum 33 angeordnete Zwischenlage 34. Die Zwischenlage 34 stellt ein feinporiges Filterelement dar, während die Außenlage 30 und die Innenlage 32 größere Porenöffnungen aufweisen können. Die Außenlage 30 und die Innenlage 32 weisen dabei die gleiche axiale Länge auf, während die Zwischenlage 34 definiert axial kürzer ausgebildet ist.

Bei dem dargestellten Blutfilterelement 10 sind die drei Lagen entlang der zwei Längsverbindungssäume 20 sowie entlang eines unteren Querverbindungssaumes 24 etwa durch Ultraschallverschweißen miteinander verbunden. Der untere Querverbindungssaum 24 ist ringförmig ausgebildet und umschließt so die untere Öffnung 17. Der Bereich mit den insgesamt drei Lagen bildet einen Filterbereich 44, welcher sich etwa über 80% der axialen Länge des Blutfilterelementes 10 von unten nach oben erstreckt.

In einem oberen Bereich ist eine Wandung des Blutfilterelementes 10 lediglich zweilagig mit der Außenlage 30 und der Innenlage 32 ausgebildet, wie anschaulich in Fig. 4 dargestellt ist. Die Außenlage 30 und die Innenlage 32 sind über einen ringförmigen oberen Querverbindungssaum 22 miteinander verschweißt, wobei der Zwischenraum 33 abgeschlossen ist. Dieser zweilagige Abschnitt bildet einen Überlaufbereich 40 in dem Blutfilterelement 10, welcher bei einem Blutrückstau nach oben aufgrund des deutlich geringeren Durchflusswiderstandes ein verbessertes Fließen des Blutes durch die Wandung hindurch ermöglicht.

An dem unteren Ende des Überlaufbereiches 40 endet die Zwischenlage 34, wobei eine Oberkante 36 der Zwischenlage 34 frei in dem Zwischenraum 33 zwischen der Außenlage 30 und der Innenlage 32 zu liegen kommt, ohne dass eine direkte fest Verbindung der Oberkante 36 mit der angrenzenden Außenlage 30 oder der angrenzenden Innenlage 32 gegeben ist.

Gemäß Fig. 5 sind zum Abschluss des unteren Filterbereiches 44 die Außenlage 30, die Innenlage 32 und die Zwischenlage 34 über den ringförmigen unteren Querverbindungssaum 24 miteinander verschweißt und fest verbunden.

Bei dem dargestellten Ausführungsbeispiel erfolgt eine Anströmung des zu reinigenden Blutes durch die obere Öffnung 15 entlang der Längsachse 12. Durch eine anliegende Druckdifferenz kann das Blut aus einem Innenraum 16 des Blutfilterelementes 10 radial nach außen strömen. Bei einem normalen Füllungsverhältnis erfolgt dies durch den dreilagigen Filterbereich 44, während bei einem eventuellen Rückstau eine radiale Durchströmung auch in dem oberen Überlaufbereich 40 erfolgen kann. Die Porenstruktur der Außenlage 30 und der Innenlage 32 sind dabei so ausgebildet, dass größere und für einen Patienten kritische Partikelgrößen und Gasbläschen rückgehalten werden, auch wenn nicht die gleiche Filter- und Reinigungswirkung wie bei einer Durchströmung in dem Filterbereich 44 mit der zusätzlichen Zwischenlage 34 erzielt wird.

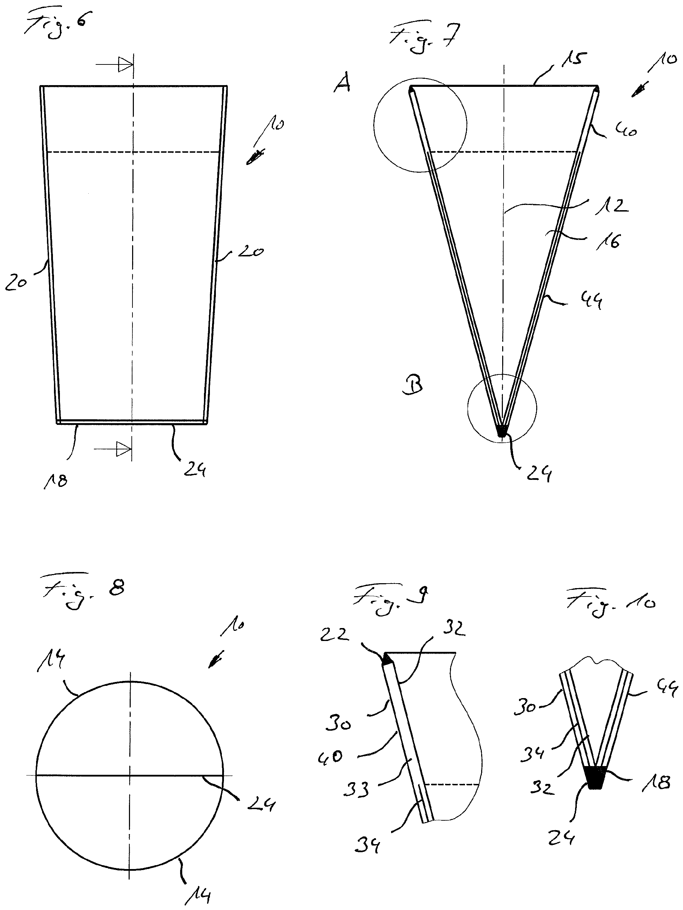

Eine weitere Ausgestaltungsmöglichkeit eines erfindungsgemäßen Blutfilterelementes 10 ist in den Figuren 6 bis 10 dargestellt. Dieses Blutfilterelement 10 weist den grundsätzlich gleichen Aufbau wie das zuvor beschriebene Blutfilterelement 10 nach den Figuren 1 bis 5 auf, wobei jedoch eine taschenartige Struktur mit einem geschlossenen Boden 18 gebildet ist. Das Blutfilterelement 10 ist dabei ebenfalls aus zwei Hälften 14 durch Übereinanderlegen und Verschweißen entlang der Längsränder mit Längsverbindungssäumen 20 hergestellt. Zusätzlich sind die Außenlage 30, die Innenlage 32 und die dazwischen angeordnete Zwischenlage 34 auch an ihren unteren Enden über einen linienförmigen unteren Querverbindungssaum 24 fest miteinander verbunden, wobei ein geschlossener Boden 18 gebildet ist.

Die Zwischenlage 34, welche das eigentliche feinporige Filterelement umfasst, erstreckt sich dabei vom unteren Ende bis zu etwa 80% der Gesamtlänge des Blutfilterelementes 10, wobei ein dreilagiger Filterbereich 44 gebildet ist. In dem restlichen zweilagigen oberen Bereich mit der Außenlage 30 und der Innenlage 32 ist ein Überlaufbereich 40 gebildet, welcher eine leichtere Durchströmung der Wandung des Blutfilterelementes 10 ermöglicht.

Auch bei diesem Ausführungsbeispiel eines Blutfilterelementes 10 erfolgt ein Anströmen des zu reinigenden Blutes von oben über eine im Wesentlichen kreisförmige Öffnung 15 in einen Innenraum 16 des Blutfilterelementes 10. Aufgrund einer anliegenden Druckdifferenz kann das Blut von innen nach außen die Wandung entweder des dreilagigen Filterbereiches 44 oder bei einer entsprechenden Füllhöhe durch den zweilagigen Überlaufbereich 40 radial nach außen erfolgen.

Am oberen Ende sind die Außenlage 30 und die Innenlage 32 unmittelbar über einen oberen Querverbindungssaum 22 miteinander verschweißt, welcher zum Bilden der oberen Öffnung ringförmig ausgebildet ist. Insgesamt wird so ein sockenartiger oder zeltförmiger Aufbau des Blutfilterelementes 10 erreicht.

## Patentansprüche

1. Blutfilterelement, welches taschen- oder schlauchförmig mit einem sich in einer Längsrichtung erstreckenden Innenraum (16) ausgebildet ist und einen mehrlagigen Aufbau aufweist, welcher eine durchlässige Außenlage (30), eine durchlässige Innenlage (32) und mindestens eine zwischen der Außenlage (30) und der Innenlage (32) angeordnete Zwischenlage (34) aufweist, welche als ein feinporiges Filterelement ausgebildet ist, wobei das Blutfilterelement (10) eine vorgegebene axiale Länge aufweist und die Außenlage (30) und die Innenlage (32) die vorgegebene axiale Länge aufweisen,
**dadurch gekennzeichnet,**
**dass** die Zwischenlage (34) axial kürzer als die Außenlage (30) und die Innenlage (32) unter Ausbildung eines Überlaufbereiches (40) ausgebildet ist, welcher an einem axialen oberen Endbereich des Blutfilterelementes (10) angeordnet ist, wobei eine Oberkante (36) der Zwischenlage (34) von der Oberkante der Außenlage (30) und der Innenlage (32) axial nach unten beabstandet ist,
**dass** die Außenlage (30), die Innenlage (32) und die Zwischenlage (34) entlang mindestens einem Längsrand unter Ausbildung eines Längsverbindungssaumes (20) fest miteinander verbunden sind und
**dass** die Außenlage (30) und die Innenlage (32) entlang ihrer Oberkante unter Ausbildung eines oberen Querverbindungssaumes (22) fest miteinander verbunden sind, wobei die Oberkante der Zwischenlage (34) frei und unverbunden zwischen der Außenlage (30) und der Innenlage (32) angeordnet ist.

2. Blutfilterelement nach Anspruch 1,
**dadurch gekennzeichnet,**
**dass** die Außenlage (30), die Innenlage (32) und/oder die Zwischenlage (34) an ihrer Unterkante unter Ausbildung eines unteren Querverbindungssaumes (24) miteinander fest verbunden sind.

3. Blutfilterelement nach Anspruch 1 oder 2,
**dadurch gekennzeichnet,**
**dass** ein Aufbau aus zwei Hälften (14) vorgesehen ist und
**dass** die zwei Hälften (14) entlang der beiden Längsränder unter Ausbildung von zwei Längsverbindungssäumen (20) fest miteinander verbunden sind.

4. Blutfilterelement nach einem der Ansprüche 1 bis 3,
**dadurch gekennzeichnet,**
**dass** eine axiale Länge des Überlaufbereiches (40) zwischen 5% bis 30% der axialen Länge des Blutfilterelementes (10) beträgt.

5. Blutfilterelement nach einem der Ansprüche 1 bis 4,
**dadurch gekennzeichnet,**
**dass** mindestens ein Verbindungssaum (20, 22, 24) durch Ultraschallverschweißen gebildet ist.

6. Blutfilterelement nach einem der Ansprüche 2 bis 5,
**dadurch gekennzeichnet,**
**dass** der untere Querverbindungssaum (24) zum Bilden eines geschlossenen Bodens (18) ausgebildet ist.

7. Blutfilterelement nach einem der Ansprüche 1 bis 6,
**dadurch gekennzeichnet,**
**dass** das Filterelement ein Filtergewebe, eine Filtermembran, ein Filtergewirk und/oder ein Filtervlies aufweist.

8. Blutfilterelement nach einem der Ansprüche 1 bis 7,
**dadurch gekennzeichnet,**
**dass** eine Öffnungs- oder Porengröße des Filterelementes zwischen 10 µm und
400 µm liegt, insbesondere zwischen 25 und 60 µm.

9. Blutfilterelement nach einem der Ansprüche 1 bis 8,
**dadurch gekennzeichnet,**
**dass** die Außenlage (30) und/oder die Innenlage (32) ausgebildet ist als ein Gewebe, Gewirk, Netz, Gitter und/oder Vlies.

10. Blutfilterelement nach einem der Ansprüche 1 bis 9,
**dadurch gekennzeichnet,**
**dass** das Material der Zwischenlage (34), der Außenlage (30) und der Innenlage (32) ein Polymermaterial ist, insbesondere Polyester oder Polyamid.

11. Blutfilterelement nach einem der Ansprüche 1 bis 10,
**dadurch gekennzeichnet,**
**dass** das Material der Zwischenlage (34), der Außenlage (30) und der Innenlage (34) gleich ist.

12. Blutfilterelement nach einem der Ansprüche 1 bis 11,
**dadurch gekennzeichnet,**
**dass** eine Öffnungsgröße der Außenlage (30) und/oder der Innenlage (32) zwischen 100 µm und 400 µm beträgt, insbesondere zwischen 150 µm und 300 µm.

13. Verfahren zum Herstellen eines Blutfilterelementes (10) nach einem der Ansprüche 1 bis 12,
**dadurch gekennzeichnet,**
**dass** die Außenlage und die Innenlage (32) an ihren Oberkanten unter Ausbildung eines oberen Querverbindungssaumes (22) fest miteinander verbunden werden, wobei die Zwischenlage (34) in einem Zwischenraum (33) zwischen der Außenlage (30) und der Innenlage (32) angeordnet ist und eine Oberkante der Zwischenlage (34) frei und ungebunden bleibt.

14. Verfahren nach Anspruch 13,
**dadurch gekennzeichnet,**
**dass** ein Verbinden der Außenlage (30), der Innenlage (32) und/oder der Zwischenlage (34) an ihren Außenrändern über ein Ultraschallverschweißen, ein thermisches Verschweißen, ein Kleben und/oder ein Heißverkleben erfolgt.

15. Verfahren nach Anspruch 13 oder 14,
**dadurch gekennzeichnet,**
**dass** durch ein Verbinden der Lagen (30, 32, 34) eine taschen- oder schlauchförmige Zwischenform mit außenliegenden Verbindungssäumen (20, 22, 24) gebildet wird und
**dass** die Zwischenform zum Bilden des Blutfilterelementes (10) gestülpt wird, wobei die Verbindungssäume (20, 22, 24) nach innen gedreht werden.
